# EUROPEAN PATENT SPECIFICATION

(11) **EP 0 311 303 B1**
(45) Date of publication and mention of the grant of the patent: **24.05.1995**
(21) Application number: 88309040.9
(22) Date of filing: 29.09.1988
(51) Int. Cl.: C07D 221/22, C07D 491/04, C07D 495/04, C07D 471/04, C07D 491/14, C07D 471/08, C07D 219/10, C07D 221/16, A61K 31/435

(54) **4-aminopyridine derivatives**
4-Aminopyridinderivate
Dérivés de la 4-aminopyridine

(30) Priority: 05.10.1987 WO PCT/US87/02546; 30.03.1988 WO PCT/US88/01070
(43) Date of publication of application: 12.04.1989
(73) Proprietor: PFIZER INC., New York, N.Y. 10017 (US)
(72) Inventor: Desai, Manoj Chandrasinhji, Mystic, Connecticut (US)
(74) Representative: Hayles, James Richard

(56) References cited:
- EP-A- 0 179 383
- EP-A- 0 268 871
- EP-A- 0 273 176
- EP-A- 0 278 499
- EP-A- 0 282 959
- EP-A- 0 287 977
- US-A- 4 550 113
- INDIAN JOURNAL OF CHEMISTRY, section B, vol. 26B, N 10, OCTOBER 1987, pp. 910-913, New Dehli, India; P. Rajamanickam et al.: "A new synthesis of dictamnine, evolitrine & 6-methyldicatamnine"

## Description

The present invention relates to 4-aminopyridine derivatives. The compounds of the present invention inhibit brain acetylcholinesterase and are useful in the treatment of Alzheimer's disease.

Tetrahydroaminoacridine, which has anticholinesterase activity, has been reported to produce improved performance in psychological tests in patients affected with Alzheimer's disease (W.K. Summers et al., The New England Journal of Medicine, 315, 1241-1245 (1986). The anticholinesterase physostigmine has also been reportedly used in the experimental treatment of Alzheimer's disease (S.D. Brinkman et al., Neurobiol. Aging, 4, 139-145 (1983).

The compounds described in the following four documents are alleged to have anticholinesterase activity:
United States Patent 4,652,567 refers to benzo(C)-1,5-naphthyridines for treating Alzheimer's disease.

United States Patent 4,631,286 refers to 9-amino-1,2,3,4-tetrahydroacridine-1-ol and related compounds for treating Alzheimer's disease.

United States Patent 4,550,113 refers to 9-amino-2,3,5,6,7,8-hexahydro-1H-cyclopenta(b)quinoline monohydrate hydrochloride for treating neuritis, injuries of the peripheral nervous system, hereditary neuromuscular diseases, and disseminated sclerosis.

United States Patent 4,578,394 refers to dihydropyridines for treating Alzheimer's disease.

United States Patent 4,540,564 refers to dihydropyridines for delivering drugs to the brain.

G.K. Patnaik et al., J. Med. Chem., 9, 483-488 (1966), refer to 4-substituted 2,3-polymethylene-quinolines having analgetic, local anesthetic, analeptic, and respiratory stimulant activities.

British Patent Specifications 1,186,061, 1,186,062, and 1,186,063 refer to benzonaphthyridine derivatives.

The present invention provides a compound of formula (I): 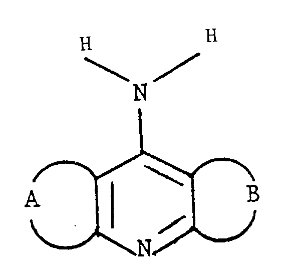
wherein A is of formula: 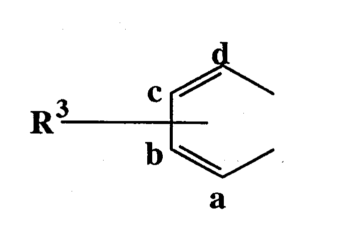
wherein the CH moiety at position a may be replaced by a nitrogen atom and R³ is H, or is fluoro and B is of formula: 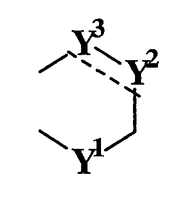
wherein the broken line between Y² and Y³ is an optional bond,
Y¹ is CH₂, O or S;
Y² is CH₂, CH, O or S and Y³ is CH₂ or CH, Y² and Y³ being CH when said optional bond is present, with the proviso that when R³ is H and there is no nitrogen at position a, only one of Y¹ and Y² may be CH₂,
at least one of Y¹, Y² and Y³ being other than CH₂, Y¹ being O or S when the optional bond is present and Y¹ and Y² being independently O or S when Y³ is CH₂,
or said compound is 9-amino-1,2,3,4-tetrahydro-1,4-methanoacridine; 9-amino-8-fluoro-1,2,3,4-tetrahydro-1,4-methanoacridine; 2,3-dihydrothieno[3,2-b]quinolin-9-amine; 2,3-dihydro-8-fluorothieno[3,2-b]quinolin-9-amine, or 1,3-dihydro-8-fluoro-thieno[3,4-b]quinolin-9-amine; or a pharmaceutically acceptable salt thereof.

Preferred compounds are those in which the CH moiety at position a is replaced by nitrogen, Y¹ is O, S or CH₂.

The present invention also relates to a pharmaceutical composition for the treatment of Alzheimer's disease comprising a compound of the formula I and to the use of compounds of the formula I in treating Alzheimer's disease. The present invention also relates to methods of preparing compounds of the formula I.

Specific preferred compounds of the present invention are the following:
9-amino-4-oxa-1,2,3,4-tetrahydroacridine;
9-amino-1,2,3,4-tetrahydro-1,4-methanoacridine;
9-amine-8-fluoro-1,2,3,4-tetrahydro-1,4-methanoacridine;
9-amino-2-oxa-1,2,3,4,-tetrahydroacridine;
9-amine-2-thia-1,2,3,4-tetrahydroacridine;
9-amino-8-fluoro-4-oxa-1,2,3,4-tetrahydroacridine;
2,3-dihydrothieno[3,2-b]quinoline-9-amine;
2,3-dihydro-8-fluoro-thieno[3,2-b]quinolin-9-amine;
1,3-dihydro-8-fluoro-thieno[3,4-b]quinolin-9-amine;
9-amino-4-thia-1,2,3,4-tetrahydroacridine; and
9-amino-8-fluoro-2-thia-1,2,3,4-tetrahydroacridine.

Other compounds of the present invention are the following:
9-amino-4,5-thiaza-1,2,3,4-tetrahydroacridine;
9-amino-1-thia-1,2,3,4-tetrahydroacridine;
9-amino-3-thia-1,2,3,4-tetrahydroacridine;
Preferred compositions of the present invention contain the foregoing preferred compounds. More preferred compositions of the present invention contain the foregoing more preferred compounds and specific preferred compounds.

Compounds of the present invention may be prepared as described below. 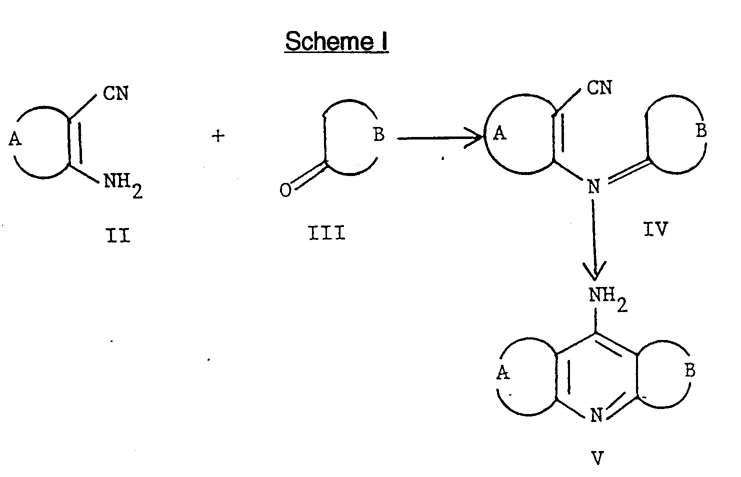
As shown in Scheme I, an aminonitrile of the formula II, wherein A is as defined above, is reacted with a ketone of the formula III wherein B is as defined above to prepare a ketimine of the formula IV. The reaction is conducted in an inert solvent, preferably an aromatic solvent (e.g., benzene or toluene), in the presence of an acid, preferably a strong acid (e.g. p-toluenesulfonic acid). The temperature should be at least about 100°C, but is otherwise not critical. Generally, the reaction is conducted at the reflux temperature of the reaction mixture, e.g., by refluxing the reaction mixture in a Dean Stark apparatus, preferably for about 6 to about 16 hours, and removing the water periodically. The reaction pressure is not critical. Generally, the reaction will be conducted at a pressure of about 0.5 to about 2 atmospheres, preferably at ambient pressure (generally, about 1 atmosphere).

The crude ketimine IV obtained after the removal of solvent is then reacted with a base (e.g., lithium diisopropylamide) in an inert solvent, preferably an anhydrous ether (e.g., tetrahydrofuran), at a temperature of about 0°C to about 25°C. The reaction pressure is not critical. Generally, the reaction will be conducted at a pressure of about 0.5 to about 2 atmospheres, preferably at ambient pressure (generally, about 1 atmosphere).

The first method works especially well when an all carbon ketone is employed in the reaction. Azeotropic removal (80°C, PTSA, 15 hours) of water from a benzene solution of norcamphor (or an all carbon ketone) and anthranilonitrile afforded the corresponding ketimine which on treatment with lithium diisopropylamide (0°C, 4 hours) in tetrahydrofuran afforded the compound of Example 1 in 25% yield. The use of this procedure is also exemplified by the preparation of the compound of Example 2.

The limitation in the general applicability of the first method is the difficulty of formation of ketimines from carbonyl compounds containing a nitrogen or oxygen atom. Titanium (IV) chloride, because of its high affinity for oxygen, has been used in the preparation of ketimines. (See H. Weingarten et al., J. Org. Chem., 32, 3246 (1967). This observation was adapted for the synethesis of 9-amino-1,2,3,4-tetrahydroacridine derivatives in a single step by the condensation of appropriate o-aminonitriles with diverse carbonyl components.

Thus, in a second method for preparing compounds of the present invention, a compound of the formula II wherein A is defined as above is reacted with a carbonyl-containing compound of the formula III wherein B is as defined above. The carbonyl-containing compound may be a ketone, a lactone, or the like. The reaction is conducted in an inert solvent in the presence of a Lewis acid (e.g., titanium (IV) chloride) and, if necessary, in the presence of a base, preferably an amine base (e.g. triethylamine). Suitable solvents include aromatic solvents (e.g., benzene or toluene) and chlorinated solvents (e.g., methylene chloride or 1,2-dichloroethane). The reaction temperature should be at least about 0°C and is preferably about 25 to about 120°C. The reaction pressure is not critical. Generally, the reaction will be conducted at a pressure of about 0.5 to about 2 atmospheres, preferably at ambient pressure (generally, about 1 atmosphere).

Thus, for example, condensation of deltavalerolactone with anthranilonitrile in methylene chloride by titanium (IV) chloride at 25°C in the presence of triethylamine (2 equivalents) afforded the compound of Example 5 (28%). This method was adopted for the synthesis of the compounds of Examples 6-13. The compounds of Examples 3 and 4 were prepared by condensing anthranilonitrile with the appropriate ketones in the presence of anhydrous zinc chloride at elevated temperature (140°C).

The compounds of Formula I are capable of forming acid addition salts with pharmaceutically acceptable acids. The acid addition salts may be prepared by reacting the base form of the appropriate compound of formula I with one or more equivalents, preferably with an excess, of the appropriate acid in an organic solvent, for example, diethyl ether or an ethanol diethyl ether mixture. Suitable acids to form these salts include the common mineral acids, e.g. hydrohalic sulfuric or phosphoric acid; the organic acids, e.g. ascorbic, citric, lactic, aspartic or tartaric acid or their aqueous solutions whose pH has been adjusted to 5.5 or less; and acids which are sparingly soluble in body fluids and which impart slow-release properties to their respective salts, e.g. pamoic or tannic acid or carboxymethyl cellulose. The preferred salt is the hydrochloride salt.

The compounds of formula I and the pharmaceutically acceptable salts thereof are useful in the treatment of various memory dysfunctions associated with decreased cholinergic function such as Alzheimer's Disease. Additionally, the compounds lead to stimulation of neuromuscular transmission, enhancement of excitation in excitable tissues (nerve, and smooth and striated muscle) as well as restoration of conductance in nerves and neuromuscular synapses in the case of injury thereof. The compounds of this invention also exhibit antidepressant activities which is particularly helpful for patients suffering from Alzheimer's Disease. The compounds of this invention are, in general, less toxic and have a broader therapeutic window than known compounds such as tacrine and physotigmine, making them therapeutically preferred.

In treating Alzhiemer's disease, the dosage of the compounds of the present invention will vary with the form of administration and the particular compound chosen. Furthermore, it will vary with the particular subject as well as the age, weight and condition of the subject under treatment as well as with the nature and extent of the symptoms. Generally, however, a dose in the range of about 1 to about 300mg/day, taken in single or divided doses, will be administered. The preferred dose is in the range of from about 1 to about 150mg/day in single or divided doses.

Generally, treatment is initiated with small dosages substantially less than the optimum dose of the compound. Thereafter, the dosage is increased by small increments until the optimum effect under the circumstances is reached.

The compounds of the present invention are used alone or in combination with pharmacologically acceptable carriers, the proportion of which is determined by the solubility and chemical nature of the compound, chosen route of administration and standard medical practice. For example, they are administered orally in the form of capsules, tablets, suspensions or solutions or they may be injected parenterally. Capsules and tablets are the preferred mode of administration. For parenteral administration, they can be used in the form of a sterile solution containing other solutes, for example, enough saline or glucose to make the solution isotonic.

Capsule and tablet compositions may contain the active ingredient in admixture with one or more pharmaceutical excipients suitable for the manufacture of capsules and tablets. Suitiable pharmaceutical excipients are, for example, starch, milk sugar, and certain types of clay. The tablets can be uncoated or they can be coated by known techniques so as to delay disintegration and absorption in the gastrointestinal tract and thereby provide a sustained action over a longer period.

Aqueous suspensions of the compounds of formula I contain the active ingredient in admixture with one or more pharmaceutical excipients suitable for the manufacture of aqueous suspensions. Suitable excipients are, for example, methylcellulose, sodium alginate, gum acacia, lecithin and so forth. Aqueous suspensions can also contain one more preservatives, one or more coloring agents, one or more flavoring agents and one or more sweetening agents.

Non-aqueous suspensions can be formulated by suspending the active ingredient in a vegetable oil for example, arachis oil, olive oil, sesame oil, or coconut oil, or in mineral oil, for example liquid paraffin, and the suspension may contain a thickening agent, for example beeswax, hard paraffin or cetyl alcohol. These compositions can also contain a sweetening agent, flavoring agent and antioxidant.

The following examples illustrate the preparation and properties of the compounds of the present invention. All melting points are uncorrected. Thin layer chromatography (TLC) was conducted on silica gel.

### Example 1

### 9-Amino-1,2,3,4-tetrahydro-1,4-methanoacridine

A solution of anthranilonitrile (3.6 g, 30.0 mmole), norcamphor (3.3 g, 30.0 mmole) and para-toluenesulfonic acid (50 mg) in benzene (50 ml) was heated to reflux using a Dean Stark apparatus. After heating for 18 hours, the reaction mixture was then cooled (25°C) and the separated water (about 1.5 ml) was withdrawn. The excess benzene was then removed under vacuum (1 mm Hg, 15 minutes). The oily residue thus obtained was dissolved in tetrahydrofuran (THF, 10 ml) and was cooled to 0°C and a solution of lithium diisopropylamide in THF (1M, 36 ml, 36 mmole) was then added. This reaction mixture was then stirred at 0°C for 3 hours. At the end of this period, the reaction mixture was quenched with 40 ml of water and was extracted with methylene chloride (200 ml). The resulting organic phase was washed with water (2 x 50 ml) and dried (anhydrous MgSO₄). The methylene chloride was removed under vacuum to afford a residue which was loaded on a silica gel flash chromatography column. Elution with 5% methanol in methylene chloride containing 1% triethylamine afforded the title compound 1.6 g (25%) as an oil which solidified on standing, m.p. 185-186°C.

### Example 2

### 9-Amino-8-fluoro-1,2,3,4-tetrahydro-1,4-methanoacridine

Following the procedure of Example 1, but substituting 2-amino-6-fluorobenzonitrile for anthranilonitrile, afforded the title compound, 23%, m.p. 173°C.

### Example 3

### 9-Amino-2-oxa-1,2,3,4-tetrahydroacridine

Anthranilonitrile (25 mmole, 2.95 g), zinc chloride (3.1 g, 25 mmole) and tetrahydro-4H-pyran-4- one were dissolved in toluene (40 ml) and heated to reflux for 2.5 days. At the end of this period, the reaction mixture was cooled (25°C), was quenched with aqueous sodium hydroxide (70 ml) and was extracted with methylene chloride (4 x 60 ml). The combined organic phases were washed with water (2 x 100 ml) and dried (anhydrous MgSO₄). The organic solvents were removed under vacuum to yield a yellow residue which was loaded on a silica gel flash chromatography column. Elution with 5% methanol in methylene chloride afforded the title compound as yellow crystals, 155 mg, 31%, m.p. 195-196°C.

### Example 4

### 9-Amino-2-thia-1,2,3,4-tetrahydroacridine

Anthranilonitrile (2.6 g, 21.5 mmole), tetrahydrothiopyran-4-one (5.0 g, 43 mmole) and zinc chloride (2.54 g, 21.5 mmole) were combined and heated to 120°C for 20 minutes. The reaction mixture was cooled and the solid residue was filtered using ethyl ether (100 ml). The resulting orange solid (5.2 g) was placed in a beaker containing a saturated solution of EDTA (ethylene diamine tetracetic acid) in water (125 ml) and the pH was adjusted to 13 with the help of 12% NaOH. The aqueous phase was then extracted with methylene chloride (4 x 50 ml) which was washed with water (2 x 70 ml) and dried (MgSO₄). Removal of methylene chloride under vacuum afforded a yellow paste (2.0 g) which was triturated with ether and filtered to afford a light yellow solid, 1.36 g, 29%, m.p. 205°C dec.

### Example 5

### 9-Amino-4-oxa-1,2,3,4-tetrahydroacridine

To a stirred solution of delta-valerolactone 1.0 g, 10.0 mmole) in methylene chloride (10 ml) at -20°C, a 1M solution of titanium (IV) chloride in methylene chloride (20 ml) was added. The reaction mixture became dark yellow in color and to it a mixture of triethylamine (2.0 g, 20 mmole) and anthranilonitrile (1.2 g, 10.0 mmole) in methylene chloride (30 ml) were added. The reaction mixture immediately became dark in color and was allowed to warm to room temperature (about 25°C) and stirred further for 15 hours. At the end of this period, the reaction mixture was treated with 25% aqueous NaOH (40 ml) and methylene chloride (100 ml) and was filtered through a 2 inch diatomaceous earth pad (Celite (trademark)) which was washed with methylene chloride (50 ml) and water (100 ml). the organic layer was separated, washed once with water (30 ml) and dried (anhydrous MgSO₄). The methylene chloride was removed under vacuum to afford an oil which was trieturated with ether to give the title compound as a white solid, 565 mg, 28%. ¹H-NMR (CDCl₃, 300 MHz, δ): 2H, m, 2.02-2.18 ppm; 2H, t, 2.63 ppm (J = 6.0 Hz); 2H, t, 4.36 ppm (J = 6.0 Hz); 2H, s, 4.64 ppm; ¹H, t, 7.25 (J = 8.0 Hz); 1H, t, 7.5 (J = 8.0 Hz); 1H, d, 7.72 (J = 8.0 Hz).

### Example 6

### 9-Amino-8-fluoro-4-oxa-1,2,3,4-tetrahydroacridine

Following the procedure of Example 5, but substituting 2-amino-6-fluorobenzonitrile for anthranilonitrile afforded the title compound 8%, m.p. 195-196°C.

### Example 7

### 2,3-Dihydrothieno[3,2-b]quinolin-9-amine

To a stirred solution of tetrahydrothiophen-3-one (1.1 g, 11 mmole) in methylene chloride (10 ml) at -78°C, a 1M solution of titanium (IV) chloride in methylene chloride (11 ml) was added. A mixture of triethylamine (2.2 g, 22 mmole) and anthranilonitrile (1.2 gms, 10.0 mmole) in methylene chloride (30 ml) was then added to the reaction mixture over a period of 5 minutes. The reaction mixture was then slowly warmed to room temperature and stirred for 2 hours. Tetrahydrothiophen-3-one (1 ml) and titanium (IV) chloride (1.0 ml) was then added to the reaction mixture and the mixture was stirred at 25°C for 16 hours. Thereafter, the reaction mixture was quenched with 12% aqueous NaOH (100 ml) and the reaction mixture was then stirred vigorously with additional methylene chloride (300 ml). The reaction mixture was then filtered through diatomaceous earth (Celite (trademark)) and the organic phase was separated. The organic solvents were removed under vacuum to afford a residue which was loaded on a flash chromatography column. Elution with 5% methanol in methylene chloride containing 1% triethylamine, afforded the title compound (1.3 gms, 64%) which was crystallized from chloroform, 560 mg, 32%, m.p. 208-210°C.

### Example 8 (not according to the invention)

### 9-Amino-6-aza-1,2,3,4-tetrahydroacridine

Titanium (IV) chloride (1.5 ml) was added to a stirred solution of 3-amino-4-cyano-pyridine (500 mg, 4.2 mmole) and cyclohexanone (0.5 ml) in 1,2-dichloroethane (15 ml). The reaction mixture was then maintained at 90°C for 12 hours. At the end of this period, cyclohexanone (2.0 ml) and 1,2-dichloroethane (5.0 ml) were added to the reaction mixture and the heating was continued for another 12 hours. Additional cyclohexanone (2.0 ml) and titanium tetrachloride (1.2 ml) were then added and the reaction mixture was maintained at 90°C for 6 hours. The reaction mixture was then cooled and was quenched with 5% aqueous NaOH (250 ml) and was stirred vigorously with methylene chloride (200 ml, 25 minutes). The reaction mixture was then treated as in Example 6 to afford the title compound (170 mg, 95% pure, 20%) after flash chromatography on silica gel (eluant:95:5:1; methylene chloride:methanol:ammonium hydroxide. This material was further purified by chromatography to afford the title compound, 75 mg, m.p. 180-181°C.

### Example 9

### 9-Amino-5-aza-1,2,3,4-tetrahydroacridine

Following the method of Example 8, but substituting 2-amino-3-cyano-pyridine for 3-amino-4-cyano-pyridine, afforded the title compound 38%, m.p. 225-228°C dec. ¹H-NMR (CDCl₃+CD₃+OD, 300 MHz, δ): 4H, bs, 1.86 ppm; 2H, bt, 2.5 ppm; 2H, bt, 2.97 ppm; 2H, vbs, 3.0-3.3 ppm; 1H, dd, 7.2 ppm (J = 8.0, 4.0 Hz); 1H, dd, 8.18 ppm (J = 8.0, 1-2 Hz); 1H, dd, 8.77 ppm (J = 4.0, 1-2 Hz).

### Example 10

### 9-Amino-4,5-oxaza-1,2,3,4-tetrahydroacridine

To a stirred solution of 2-amino-3-cyano-pyradine (360 mg, 30 mmole) and delta-valerolactone (360 mg, 3.6 mmole) in 1,2-dichloroethane (7.0 ml), titanium (IV) chloride (0.9 ml) was added and then the reaction mixture was maintained at 90°C for 18 hours. The reaction mixture was then quenched with 15% aqueous NaOH (200 ml) and was stirred vigorously with methylene chloride (200 ml, 25 minutes). The reaction mixture was then worked up as in example 15 to afford the title compound, 8%, m.p. 269-270°C dec.

### Example 11

### 9-Amino-4-thia-1,2,3,4-tetrahydroacridine

Following the method of Example 5, but substituting delta-thiovalerolactone for delta-valerolactone afforded the title compound, 4%, m.p. 190°C.

### Example 12

### 9-Amino-8-fluoro-2-thia-1,2,3,4-tetrahydroacridine

Following the method of Example 5, but substituting tetrahydrothiopyran-4-one for delta-valerolactone and 2-amino-6-fluorobenzonitrile for anthranilonitrile, afforded the title compound, 19%, m.p. 175-176°C.

### Example 13

### 2,3-Dihydro-8-fluorothieno[3,2-b]quinolin-9-amine

### (Compound A) and 1,3-Dihydro-8-fluoro-thieno[3,4-b]quinolin-9-amine (Compound B)

Following the method of Example 7 but substituting 2-amino-6-fluorobenzonitrile for anthranilonitrile afforded a 1:1 mixture of the two title compounds. Compound A, m.p. 137°C, Compound B (m.p. 198°C dec.): ¹H-NMR (CDCl₃, 300 MHz,): 2H, s, 4.09 ppm; 2H, s, 4.38 ppm; 2H, bs, 5.3 ppm; 1H, dd, 7.0 ppm (J = 7.3, 14.5 Hz): 1H, dd, 7.47 ppm(J = 7.3, 10.5 Hz); 1H, d, 7.68 ppm (J = 10.5 Hz).

### Example 14

The ability of the title compounds of Examples 1-7 and 8-13 to inhibit brain acetyl-cholinesterase was determined by the spectrophotometric method of G.L. Ellman et a. (Biochemical Pharmacology, 7, 88 (1961)). All of the compounds had IC₅₀ (molar) values between 5 »M and 0.1»M.

## Claims (Claims for the following Contracting State(s): AT, BE, CH, DE, FR, GB, IT, LI, LU, NL, SE)

1. A compound of the formula (I): 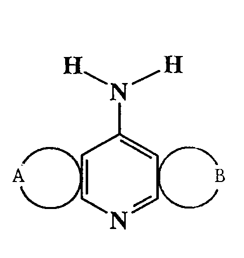 wherein A is of formula: 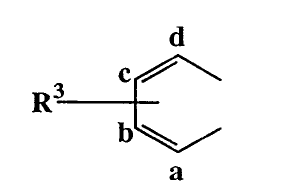 wherein the CH moiety at position a may be replaced by a nitrogen atom and R³ is H, or is fluoro
and B is of formula: 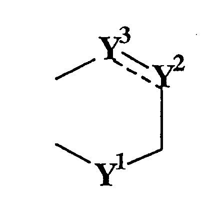 wherein the broken line between Y² and Y³ is an optional bond,
Y¹ is CH₂, O or S;
Y² is CH₂, CH, O or S and Y³ is CH₂ or CH, Y² and Y³ being CH when said optional bond is present,
with the proviso that when R³ is H and there is no nitrogen at position a, only one of Y¹ and Y² may be CH₂,
at least one of Y¹, Y² and Y³ being other than CH₂, Y¹ being O or S when the optional bond is present and Y¹ and Y² being independently O or S when Y³ is CH₂,
or said compound is 9-amino-1,2,3,4-tetrahydro-1,4-methanoacridine; 9-amino-8-fluoro-1,2,3,4-tetrahydro-1,4-methanoacridine; 2,3-dihydrothieno[3,2-b]quinolin-9-amine; 2,3-dihydro-8-fluorothieno[3,2-b]quinolin-9-amine, or 1,3-dihydro-8-fluoro-thieno[3,4-b]quinolin-9-amine;
or a pharmaceutically acceptable salt thereof.

2. A compound according to claim 1, in which the CH moiety at position a is replaced by nitrogen, Y¹ is O, S or CH₂ and Y² is CH₂.

3. A compound according to claim 1 or 2, in admixture with a pharmaceutically acceptable diluent or carrier.

4. A compound according to claim 1 or 2, for use in medicine.

5. Use of a compound according to claim 1 or 2 for making a medicament for treatment of Alzheimer's disease.

## Claims (Claims for the following Contracting State(s): ES, GR)

1. A process for preparing a compound of formula (I): 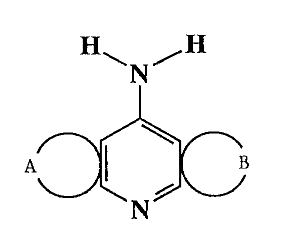 wherein A is of formula: 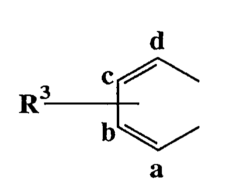 wherein the CH moiety at position a may be replaced by a nitrogen atom and R³ is H, or is fluoro
and B is of formula: 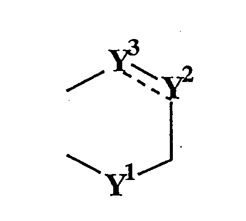 wherein the broken line between Y² and Y³ is an optional bond,
Y¹ is CH₂, O or S;
Y² is CH₂, CH, O or S and Y³ is CH₂ or CH, Y² and Y³ being CH when said optional bond is present,
with the proviso that when R³ is H and there is no nitrogen at position a, only one of Y¹ and Y² may be CH₂,
at least one of Y¹, Y² and Y³ being other than CH₂, Y¹ being O or S when the optional bond is present and Y¹ and Y² being independently O or S when Y³ is CH₂,
or said compound is 9-amino-1,2,3,4-tetrahydro-1,4-methanoacridine; 9-amino-8-fluoro-1,2,3,4-tetrahydro-1,4-methanoacridine; 2,3-dihydrothieno[3,2-b]quinolin-9-amine; 2,3-dihydro-8-fluorothieno[3,2-b]quinolin-9-amine, or 1,3-dihydro-8-fluoro-thieno[3,4-b]quinolin-9-amine;
or a pharmaceutically acceptable salt thereof; characterized by cyclization of a ketimine of formula: 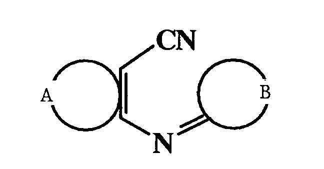 where A and B are defined above, or A and B are the following pairs of groups respectively: 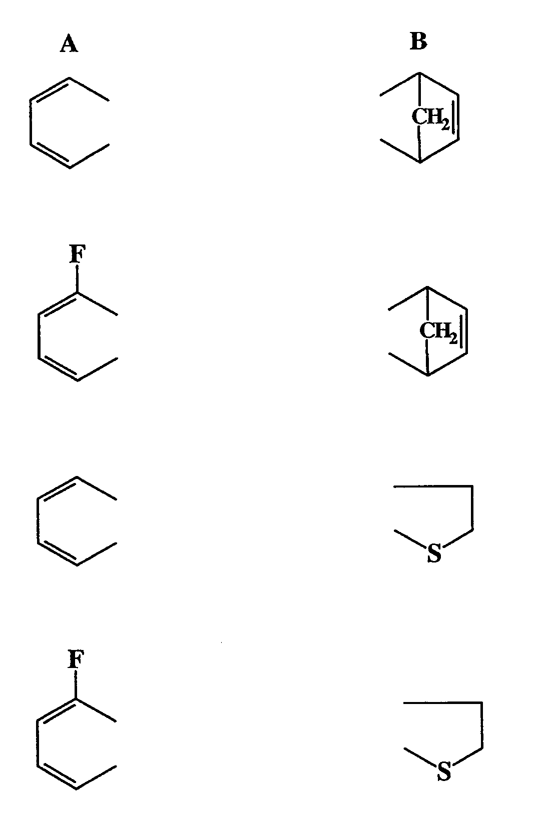 and, if necessary, allowing the product to react with a pharmaceutically acceptable acid.

2. A process according to claim 1, in which the CH moiety at position a is replaced by nitrogen, Y¹ is O, S or CH₂ and Y² is CH₂.

## Patentansprüche (Patentansprüche für folgende(n) Vertragsstaat(en): AT, BE, CH, DE, FR, GB, IT, LI, LU, NL, SE)

1. Verbindung der Formel (I), 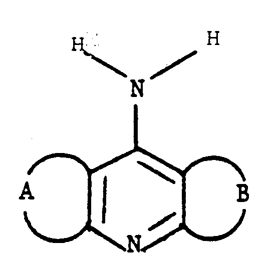 worin A die Formel hat: 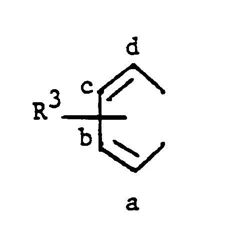 worin der CH Teil an einer Stellung a durch ein Stickstoffatom ersetzt sein kann und R³ H oder Fluor ist und B die Formel hat: 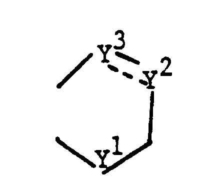 worin die gestrichelte Linie zwischen Y² und Y³ eine fakultative Bindung bedeutet,
Y¹ CH₂, O oder S ist,
Y² CH₂, CH, O oder S ist und Y³ CH₂ oder CH ist, wobei Y² und Y³ CH sind, wenn diese fakultative Bindung vorliegt, mit der Maßgabe, daß, wenn R³ H ist und kein Stickstoffatom in der Stellung a vorliegt, nur eines von Y¹ und Y² CH₂ sein kann,
mindestens eines von Y¹, Y² und Y³ eine andere Bedeutung als CH₂ hat, Y¹ O oder S ist, wenn die fakultative Bindung vorliegt, und Y¹ und Y² unabhängig O oder S sind, wenn Y³ CH₂ ist,
oder die Verbindung 9-Amino-1,2,3,4-tetrahydro-1,4-methanoacridin, 9-Amino-8-fluor-1,2,3,4-tetrahydro-1,4-methanoacridin, 2,3-Dihydrothieno[3,2-b]chinolin-9-amin, 2,3-Dihydro-8-fluorthieno[3,2-b]chinolin-9-amin oder 1,3-Dihydro-8-fluorthieno[3,4-b]chinolin-9-amin ist, oder ein pharmazeutisch annehmbares Salz davon.

2. Verbindung nach Anspruch 1, in der der CH Teil in Stellung a durch Stickstoff ersetzt ist, Y¹ O, S oder CH₂ ist und Y³ CH₂ ist.

3. Verbindung nach Anspruch 1 oder 2 in Mischung mit einem pharmazeutisch annehmbaren Verdünnungsmittel oder Träger.

4. Verbindung nach Anspruch 1 oder 2 zur Verwendung in der Medizin.

5. Verwendung einer Verbindung nach Anspruch 1 oder 2 zur Herstellung eines Arzneimittels für die Behandlung der Alzheimer'schen Krankheit.

## Patentansprüche (Patentansprüche für folgende(n) Vertragsstaat(en): ES, GR)

1. Verfahren zur Herstellung einer Verbindung der Formel (I), 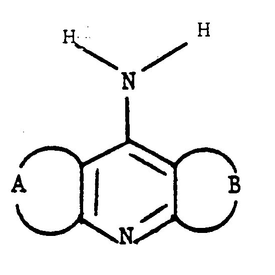 worin A die Formel hat: 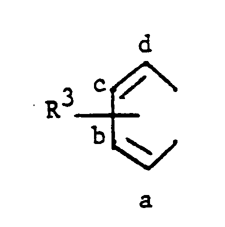 worin der CH Teil an einer Stellung a durch ein Stickstoffatom ersetzt sein kann und R³ H oder Fluor ist und B die Formel hat: 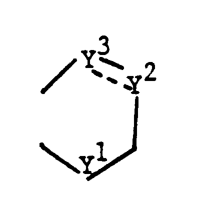 worin die gestrichelte Linie zwischen Y² und Y³ eine fakultative Bindung bedeutet,
Y¹ CH₂, O oder S ist,
Y² CH₂, CH, O oder S ist und Y³ CH₂ oder CH ist, wobei Y² und Y³ CH sind, wenn diese fakultative Bindung vorliegt, mit der Maßgabe, daß, wenn R³ H ist und kein Stickstoffatom in der Stellung a vorliegt, nur eines von Y¹ und Y² CH₂ sein kann,
mindestens eines von Y¹, Y² und Y³ eine andere Bedeutung als CH₂ hat, Y¹ O oder S ist, wenn die fakultative Bindung vorliegt, und Y¹ und Y² unabhängig O oder S sind, wenn Y³ CH₂ ist,
oder die Verbindung 9-Amino-1,2,3,4-tetrahydro-1,4-methanoacridin, 9-Amino-8-fluor-1,2,3,4-tetrahydro-1,4-methanoacridin, 2,3-Dihydrothieno[3,2-b]chinolin-9-amin, 2,3-Dihydro-8-fluorthieno[3,2-b]chinolin-9-amin oder 1,3-Dihydro-8-fluorthieno[3,4-b]chinolin-9-amin ist, oder ein pharmazeutisch annehmbares Salz davon,
gekennzeichnet durch das Cyclisieren eines Ketimins der Formel 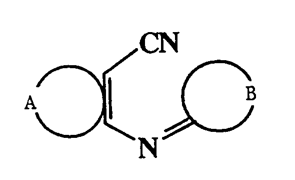 worin A und B wie oben definiert sind oder A und B die folgenden Paare von Gruppen sind: 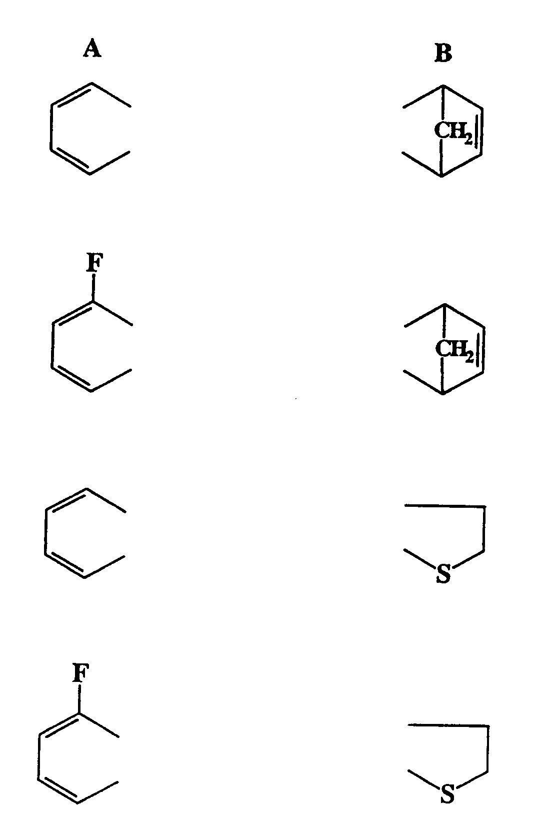 und, falls nötig, Reagierenlassen des Produktes mit einer pharmazeuztisch annehnmbaren Säure.

2. Verfahren nach Anspruch 1, bei dem der CH Teil in Stellung a durch Stickstoff ersetzt ist, Y¹ O, S oder CH₂ ist und Y² CH₂ ist.

## Revendications (Revendications pour l'(les) Etat(s) contractant(s) suivant(s): AT, BE, CH, DE, FR, GB, IT, LI, LU, NL, SE)

1. Composé de formule (I) : 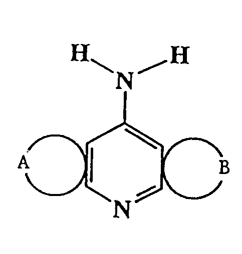 dans laquelle A répond à la formule : 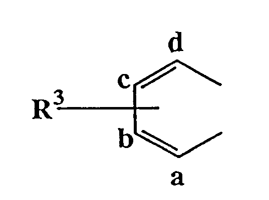 dans laquelle le groupement CH en position a peut être remplacé par un atome d'azote et R³ représente H ou représente un groupe fluoro,
et B répond à la formule : 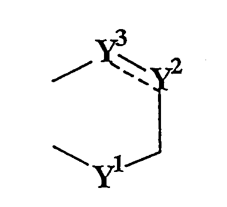 dans laquelle la ligne discontinue entre Y² et Y³ représente une liaison facultative,
Y¹ représente un groupe CH₂, O ou S ;
Y² représente un groupe CH₂, CH, O ou S et Y³ représente un groupe CH₂ ou CH, Y² et Y³ représentant des groupes CH lorsque ladite liaison facultative est présente,
sous réserve que, lorsque R³ représente H et qu'il n'existe aucun atome d'azote en position a, un seul des groupes Y¹ et Y² puisse représenter un groupe CH₂,
au moins un des groupes Y¹, Y² et Y³ étant autre qu'un groupe CH₂, Y¹ représente O ou S lorsque la liaison facultative est présente, et Y¹ et Y² représentant indépendamment O ou S lorsque Y³ représente un groupe CH₂,
ou bien ledit composé consiste en la 9-amino-1,2,3,4-tétrahydro-1,4-méthanoacridine ; la 9-amino-8-fluoro-1,2,3,4-tétrahydro-1,4-méthanoacridine ; la 2,3-dihydrothiéno[3,2-b]quinoléine-9-amine ; la 2,3-dihydro-8-fluorothiéno[3,2-b]quinoléine-9-amine ; ou la 1,3-dihydro-8-fluorothiéno[3,4-b]quinoléine-9-amine ;
ou un de ses sels pharmaceutiquement acceptables.

2. Composé suivant la revendication 1, dans lequel le groupe CH en position a est remplacé par un atome d'azote, Y¹ représente O, S ou un groupe CH₂, et Y² représente un groupe CH₂.

3. Composé suivant la revendication 1 ou 2, en mélange avec un diluant ou support pharmaceutiquement acceptable.

4. Composé suivant la revendication 1 ou 2, destiné à être utilisé en médecine.

5. Utilisation d'un composé suivant la revendication 1 ou 2 pour la préparation d'un médicament destiné au traitement de la maladie d'Alzheimer.

## Revendications (Revendications pour l'(les) Etat(s) contractant(s) suivant(s): ES, GR)

1. Procédé de préparation d'un composé de formule (I) : 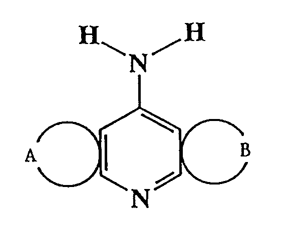 dans laquelle A répond à la formule : 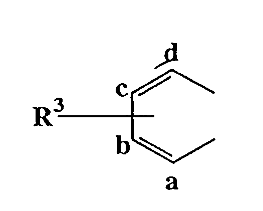 dans laquelle le groupement CH en position a peut être remplacé par un atome d'azote et R³ représente H ou représente un groupe fluoro, et B répond à la formule : 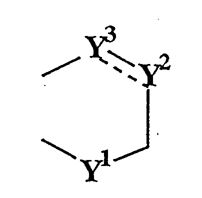 dans laquelle la ligne discontinue entre Y² et Y³ représente une liaison facultative,
Y¹ représente un groupe CH₂, O ou S ;
Y² représente un groupe CH₂, CH, O ou S et Y³ représente un groupe CH₂ ou CH, Y² et Y³ représentant des groupes CH lorsque ladite liaison facultative est présente,
sous réserve que, lorsque R³ représente H et qu'il n'existe aucun atome d'azote en position a, un seul des groupes Y¹ et Y² puisse représenter un groupe CH₂,
au moins un des groupes Y¹, Y² et Y³ étant autre qu'un groupe CH₂, Y¹ représentant O ou S lorsque la liaison facultative est présente, et Y¹ et Y² représentant indépendamment O ou S lorsque Y³ représente un groupe CH₂,
ou dudit composé consistant en 9-amino-1,2,3,4-tétrahydro-1,4-méthanoacridine ; 9-amino-8-fluoro-1,2,3,4-tétrahydro-1,4-méthanoacridine ; 2,3-dihydrothiéno[3,2-b]quinoléine-9-amine ; 2,3-dihydro-8-fluorothiéno[3,2-b]quinoléine-9-amine ; ou 1,3-dihydro-8-fluorothiéno[3,4-b]quinoléine-9-amine ; ou bien d'un de ses sels pharmaceutiquement acceptables ;
caractérisé par la cyclisation d'une cétimine de formule 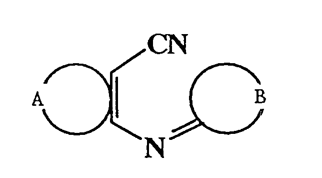 dans laquelle A et B répondent aux définitions précitées, ou bien A et B représentent respectivement les paires de groupes suivantes 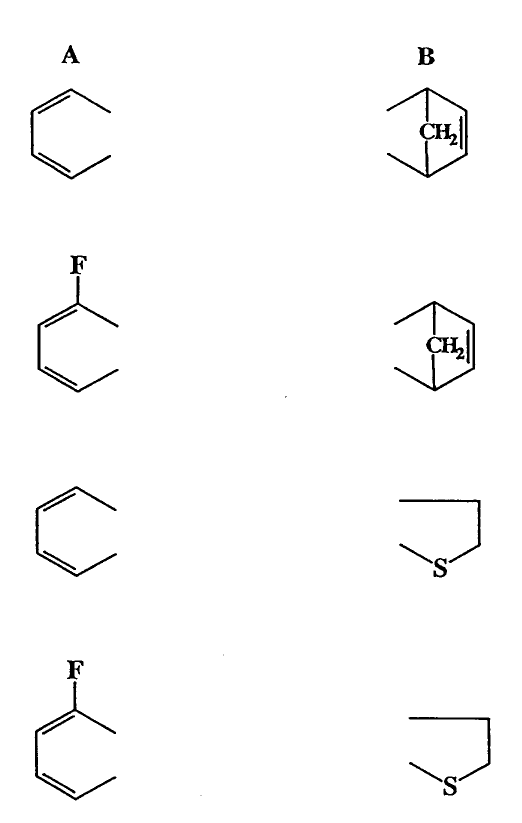 et, si nécessaire, l'étape consistant à laisser le produit réagir avec un acide pharmaceutiquement acceptable.

2. Procédé suivant la revendication 1, dans lequel le groupement CH en position a est remplacé par un atome d'azote, Y¹ représente O, S ou un groupe CH₂, et Y² représente un groupe CH₂.
